# EUROPEAN PATENT APPLICATION

(11) **EP 4 775 217 A1**
(43) Date of publication of application: **15.07.2026**
(21) Application number: 24853035.4
(22) Date of filing: 30.10.2024
(51) Int. Cl.: A61K 38/24, A61P 15/08, A61P 5/24

(54) **COMPOSITION COMPRISING BETA CORE FRAGMENT HCG AS ACTIVE INGREDIENT FOR TREATING INFERTILITY**

(30) Priority: 24.10.2024 KR 20240146770
(71) Applicant: Adtech Co., Ltd., Gunpo-si, Gyeonggi-do 15845 (KR)
(72) Inventor: CHANG, Jin Dong, Seoul 03165 (KR); KWAG, Won Jae, Hwaseong-si, Gyeonggi-do 18374 (KR); CHO, Sung Jin, Cheonan-si, Chungcheongnam-do 31112 (KR); LEE, Jae Ho, Yongin-si, Gyeonggi-do 16848 (KR)
(74) Representative: dompatent
(86) International application number: PCT/KR2024/016852
(87) International publication number: WO 2026/089099

(57) **Abstract**

The present disclosure relates to a composition for treating infertility, which includes beta core fragment hCG (βcf hCG) as an active ingredient. The βcf hCG is identified to stimulate endometrial development in early pregnancy, is highly expressed in placental tissue, and is involved in maintaining fetal development in early pregnancy. The composition plays an important role in implantation and maintenance of early pregnancy by increasing the thickness of the endometrium, increasing the expression of genes necessary for uterine development, and promoting the growth of uterine-derived cell line. The composition can be usefully used to treat habitual miscarriage in female infertility, and to increase the success rate of pregnancy by improving the implantation rate of fertilized eggs in the process of in vitro fertilization.

## Description

### [TECHNICAL FIELD]

The present disclosure relates to a composition for treating infertility including beta core fragment hCG (βcf hCG) as an active ingredient.

### [BACKGROUND ART]

Recently, with the aging society, the age of mothers giving birth is increasing, and infertility is increasing. In addition, the pregnancy rate is decreasing significantly due to the increase in stress women receive due to environmental pollution caused by industrialization and women's social advancement. Commonly known causes of female infertility include ovulation disorders, transport disorders of fertilized eggs, and implantation disorders. Most infertility problems caused by ovulation disorders and transport disorders of fertilized eggs are solved through in vitro fertilization (IVF), but there is still no clear solution for infertility caused by implantation disorders.

In order to maintain a successful pregnancy, embryo development, endometrial development, implantation, placental formation, hormonal control, and exchange of nutrients and gases between the mother and the fetus are necessary. In the early stages of pregnancy, the fetus undergoes morphological changes from a spherical blastocyst to a filamentous shape to implant in the uterine lining, and forms a successful impermeable placenta.

Implantation is a phenomenon in early pregnancy in which a fertilized blastocyst that is developing into an embryo attaches to the endometrial layer of the mother. The uterus must maintain an endometrial thickness of at least about 12 mm during the fertile period for successful pregnancy, and the uterus needs to continuously expand and normal cell growth needs to occur during pregnancy. This endometrial development needs to occur well to maintain pregnancy without miscarriage. To date, specific regulatory factors in the process of implantation and endometrial development have not been clearly identified.

Meanwhile, human chorionic gonadotropin (hCG) is a glycoprotein hormone produced during pregnancy. hCG is produced in the trophoblast of the placenta and continues to produce progesterone in early pregnancy, thereby maintaining implantation until the 10th week of pregnancy when placental function is complete.

The active form in the body, full-form hCG (I-hCG), has a molecular weight of about 37 kDa and consists of 244 amino acids. I-hCG is largely composed of two subunits, alpha (α) and beta (β), and the alpha subunit contains 92 amino acids, and the beta subunit contains 145 amino acids. I-hCG is generally present in the blood during pregnancy and is the main structure that exhibits biological activity. In addition to the above-mentioned active form, various dissociation and decomposition products of hCG are found in blood and urine, and they exist as nicked hCG (N-hCG), free beta hCG (free β-hCG), free alpha hCG (free α-hCG), and beta core fragment hCG (βcf hCG).

βcf hCG is an hCG protein that mainly exists in pregnant women's urine and has a molecular weight of approximately 14 kDa. At the 4th week of LMP (Last menstrual period; the last menstrual period), I-hCG is predominantly secreted, and from the 5th week of LMP, βcf hCG begins to gradually increase and is secreted at a concentration that is predominant over I-hCG on the 5th week and 6th day or after the 6th week. In a previous study by the present inventors, it is revealed that the above-mentioned βcf hCG can be used as a marker for pregnancy diagnosis.

Against this background, the present inventors studies a specific maintenance factor for the implantation of an early fertilized egg in the uterus, and identifies that beta core fragment hCG (βcf hCG) is necessary and critical factor for early pregnancy and maintenance, thereby completing the present disclosure.

### [Prior art document]

### [Non-patent document]

(Non-patent document 0001) Spencer TE et al., Reproduction, 2004, 128(6):657-668.

### [DETAILED DESCRIPTION OF THE INVENTION]

### [TECHINICAL PROBLEM]

The present disclosure is to provide a pharmaceutical composition for preventing or treating infertility, which includes beta core fragment hCG (βcf hCG) as an active ingredient.

The present disclosure is also to provide a method for preventing or treating infertility, which includes administering a pharmaceutical composition to a subject.

The present disclosure is also to provide a pharmaceutical composition for promoting pregnancy, which includes beta core fragment hCG (βcf hCG) as an active ingredient.

### [TECHNICAL SOLUTION]

Advantages and features of the inventive concept and methods for achieving them will be apparent with reference to embodiments described below in detail in conjunction with the accompanying drawings. However, the inventive concept is not limited to the embodiments disclosed below, but can be implemented in various forms, and these embodiments are to make the disclosure of the inventive concept complete, and are provided so that this disclosure will be thorough and complete and will fully convey the scope of the invention to those of ordinary skill in the art, which is to be defined only by the scope of the claims.

The terminology used herein is for the purpose of describing particular embodiments only and is not intended to be limiting of the inventive concept. The singular expressions include plural expressions unless the context clearly dictates otherwise. In this specification, the terms "comprises" and/or "comprising" are intended to specify the presence of stated elements, but do not preclude the presence or addition of elements. Like reference numerals refer to like elements throughout the specification, and "and/or" includes each and all combinations of one or more of the mentioned elements. Although "first", "second", and the like are used to describe various components, these components are of course not limited by these terms. These terms are only used to distinguish one component from another. Thus, a first element discussed below could be termed a second element without departing from the teachings of the inventive concept.

Unless otherwise defined, all terms (including technical and scientific terms) used herein have the same meaning as commonly understood by one of ordinary skill in the art to which this invention belongs. It will be further understood that terms such as those defined in commonly used dictionaries, will not be interpreted in an idealized or overly formal sense unless expressly so defined herein.

The present disclosure provides a pharmaceutical composition for preventing or treating infertility, which includes beta core fragment hCG (βcf hCG) as an active ingredient.

The human chorionic gonadotropin (hCG) of the present disclosure is a glycoprotein hormone produced during pregnancy. hCG is produced in the trophoblast of the placenta and continues to produce progesterone in the early stage of pregnancy, thereby maintaining implantation until the 10th week of pregnancy when the placental function is completed.

The full-form hCG (I-hCG), which is an active form in the body, has a molecular weight of about 37 kDa and is composed of 244 amino acids. I-hCG is largely composed of two subunits, alpha (α) and beta (β), and the alpha subunit (α-hCG) contains 92 amino acids, and the beta subunit (β-hCG) contains 145 amino acids.

I-hCG is generally present in blood during pregnancy and is the main biologically active structure. In addition to the active form, various dissociation and decomposition products of hCG are found in blood and urine, and they exist as nicked hCG (N-hCG), free beta hCG (free β-hCG), free alpha hCG (free α-hCG), beta core fragment hCG (βcf hCG), and the like.

The beta core fragment hCG (βcf hCG) of the present disclosure is an hCG protein that mainly exists in the urine of pregnant women and has a molecular weight of about 14 kDa. I-hCG is secreted predominantly at 4 weeks of LMP (Last menstrual period), and βcf hCG begins to gradually increase from 5 weeks of LMP and is secreted at a concentration that is superior to I-hCG at 5 weeks and 6 days or after 6 weeks.

The beta core fragment hCG of the present disclosure includes amino acids 6-40 and 55-92 of β-hCG. The amino acid sequence of the β-hCG includes sequence number 1:

Sequence number 1: Ser Lys Glu Pro Leu Arg Pro Arg Cys Arg Pro Ile Asn Ala Thr Leu Ala Val Glu Lys Glu Gly Cys Pro Val Cys Ile Thr Val Asn Thr Thr Ile Cys Ala Gly Tyr Cys Pro Thr Met Thr Arg Val Leu Gln Gly Val Leu Pro Ala Leu Pro Gln Val Val Cys Asn Tyr Arg Asp Val Arg Phe Glu Ser Ile Arg Leu Pro Gly Cys Pro Arg Gly Val Asn Pro Val Val Ser Tyr Ala Val Ala Leu Ser Cys Gln Cys Ala Leu Cys Arg Arg Ser Thr Thr Asp Cys Gly Gly Pro Lys Asp His Pro Leu Thr Cys Asp Asp Pro Arg Phe Gln Asp Ser Ser Ser Lys Ala Pro Pro Pro Ser Leu Pro Ser Pro Ser Arg Leu Pro Gly Pro Ser Asp Thr Pro Ile Leu Pro Gln

(SKEPLRPRCRPINATLAVEKEGCPVCITVNTTICAGYCPTMTRVLQG
VLPALPQVVCNYRDVRFESIRLPGCPRGVNPVVSYAVALSCQCALCRRSTT
DCGGPKDHPLTCDDPRFQDSSSSKAPPPSLPSPSRLPGPSDTPILPQ).

The amino acid sequence of the beta core fragment hCG includes sequence numbers 2 and 3:
Sequence number 2: Arg Pro Arg Cys Arg Pro Ile Asn Ala Thr Leu Ala Val Glu Lys Glu Gly Cys Pro Val Cys Ile Thr Val Asn Thr Thr Ile Cys Ala Gly Tyr Cys Pro Thr
   (RPRCRPINATLAVEKEGCPVCITVNTTICAGYCPT),
Sequence number 3: Val Val Cys Asn Tyr Arg Asp Val Arg Phe Glu Ser Ile Arg Leu Pro Gly Cys Pro Arg Gly Val Asn Pro Val Val Ser Tyr Ala Val Ala Leu Ser Cys Gln Cys Ala Leu
   (VVCNYRDVRFESIRLPGCPRGVNPVVSYAVALSCQCAL).

The beta core fragment hCG of the present disclosure can be secreted from the placenta and the corpus luteum of the ovary.

The placenta of the present disclosure is an organ that transfers nutrients and oxygen between the mother and the fetus during pregnancy and discharges waste products. It is formed from the early stage of pregnancy and is created by combining the endometrium of the mother and the chorion of the fetus. The placenta is responsible for blood exchange between the fetus and the mother and plays an important role in maintaining pregnancy by secreting hormones (estrogen, progesterone, hCG).

The corpus luteum of the present disclosure is a temporary endocrine organ formed in the ovary after ovulation. The remaining part of the follicle is transformed when ovulated to form the corpus luteum, and mainly secretes progesterone and a small amount of estrogen. This hormone helps the fertilized egg to implant and plays an important role in maintaining pregnancy.

In a specific embodiment of the present disclosure, the beta core fragment hCG is very specifically expressed in placental tissue and is identified to be associated with changes in the expression of epithelial granular tissue involved in implantation of a fertilized egg in the endometrial tissue of a non-pregnant uterus. This indicates that it stimulates endometrial development in the early stage of pregnancy and is involved in maintaining fetal development.

The composition of the present disclosure can be administered to a subject before embryo implantation, specifically, it can be administered within 50 to 90 hours before implantation, and more specifically, it can be administered within 60 to 80 hours.

Administration of the composition of the present disclosure can include intrauterine administration, but is not limited thereto.

Intrauterine administration of the present disclosure is an administration method used for infertility treatment, assisted reproductive technology (IVF), or treatment of specific uterine diseases by directly injecting it into the uterus. Since the pharmaceutical composition is directly delivered to the endometrium by the administration method, more effective treatment is possible locally.

The composition of the present disclosure can increase the thickness of the endometrium.

In a specific embodiment of the present disclosure, as a result of treating the composition containing the beta core fragment hCG, the thickness of the endometrium is identified to increase by 19.72% on the first day after administration compared to the control group (saline administration group) and to increase by 23.03% on the third day after administration. In addition, it is identified to increase compared to the I-hCG administration group. This indicates that the beta core fragment hCG plays a critical role in increasing the thickness of the endometrium.

The composition of the present disclosure can promote the proliferation of endometrial cells.

In a specific embodiment of the present disclosure, when the composition containing the beta core fragment hCG is treated for 24 hours to cell lines derived from placenta and uterine tissue, it is identified that cell growth increased.

The composition of the present disclosure can increase the expression of a gene related to uterine receptive development and implantation.

The gene may include, but is not limited to, HOXA10, HOXA11, DEDD, Cyclin D3, CDK4/6, EGR, PIGF, HAND2 or MSX1.

HOXA10 plays a role in improving endometrial receptivity by regulating cholesterol synthesis in endometrial stromal cells. HOXA11 is involved in endometrial proliferation and differentiation and embryonic development. DEDD plays an essential role in forming a uterine environment suitable for early pregnancy. Cyclin D3 and CDK4/6 are closely related to cell cycle regulation for endometrial cell proliferation. HAND2 plays an important role in forming a proper implantation environment for pregnancy, and forms a uterine environment that is sensitized to progesterone when implantation begins. PIGF affects the implantation and development of embryos and the development of the endometrium. EGR is an immediate early gene that is important for cell growth and differentiation, and is expressed in response to stimulation rapidly. MSX1 controls uterine receptivity by regulating uterine epithelial function at the time of embryo implantation.

The infertility of the present disclosure may be at least one selected from the group consisting of nonimplantation of ovum, female infertility associated with anovulation, female infertility of tubal origin, female infertility of uterine origin, female infertility of cervical origin, and female infertility associated with male factors, but is not limited thereto.

The pharmaceutical composition of the present disclosure may include beta core fragment hCG as an active ingredient, and may additionally include at least one active ingredient having the same or similar function, that is, having an effect of treating infertility, in addition to the active ingredient.

The term pharmaceutical composition of the present disclosure may be described interchangeably with pharmaceutical composition.

The pharmaceutical composition of the present disclosure may further include a pharmaceutically acceptable additive.

The additive may further include a suitable carrier, excipient, and diluent commonly used in the manufacture of pharmaceutical composition.

The pharmaceutical composition of the present disclosure may include a pharmaceutically acceptable carrier. The pharmaceutically acceptable carrier is one commonly used in the preparation of a pharmaceutical composition, and includes lactose, dextrose, sucrose, sorbitol, mannitol, xylitol, erythritol, maltitol, starch, arabia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil, but is not limited thereto.

The pharmaceutical composition of the present disclosure can be formulated and used in the form of oral formulations such as powders, granules, tablets, capsules, suspensions, emulsions, syrups, aerosols, external preparations, suppositories, or sterile injection solutions, respectively, according to conventional methods. Specifically, when formulated, it can be prepared using diluents or excipients such as fillers, weighting agents, binders, wetting agents, disintegrants, and surfactants that are commonly used.

In addition to the above components, the pharmaceutical composition of the present disclosure may additionally include lubricants, wetting agents, sweeteners, flavoring agents, emulsifiers, suspending agents, preservatives, and the like.

The pharmaceutical composition of the present disclosure can be administered parenterally, and specifically, can be administered by intrauterine injection, but is not limited thereto.

The appropriate dosage of the pharmaceutical composition of the present disclosure varies depending on factors such as the formulation method, administration method, patient's age, weight, sex, pathological condition, food, administration time, administration route, excretion rate and response sensitivity, and the degree of cancer in the patient, and can be appropriately selected by a person skilled in the art.

The pharmaceutical composition of the present disclosure can be manufactured in a unit dosage form or can be manufactured by putting it into a multi-dose container by formulating it using a pharmaceutically acceptable carrier and/or excipient according to a method that can be easily performed by a person skilled in the art to which the present disclosure pertains. At this time, the formulation can be in the form of a solution, suspension, or emulsion in an oil or aqueous medium, or in the form of an extract, powder, granule, tablet, or capsule, and can additionally include a dispersant or stabilizer.

The subject of the administration of the present disclosure includes, but is not limited to, a human, a monkey, a cow, a horse, a pig, a sheep, a chicken, a cat, a dog, a mouse, a rabbit, and the like.

In addition, the present disclosure provides a method for preventing or treating infertility, which includes administering the pharmaceutical composition to a subject suspected of or diagnosed with infertility.

The terms, pharmaceutical composition, and infertility of the present disclosure are as described above.

The treatment method of the present disclosure includes administering the pharmaceutical composition of the present disclosure in a pharmaceutically effective amount to a subject suspected of or diagnosed with infertility. The subject refers to all mammals including dogs, cows, horses, rabbits, mice, rats, chickens, or humans, but the subject of the present disclosure is not limited by the above examples.

The pharmaceutical composition may be administered parenterally, and may be administered by a suitable method including intrauterine administration, if necessary, for local treatment. The preferred dosage of the pharmaceutical composition of the present disclosure varies depending on the condition and weight of the subject, the degree of the disease, the drug form, the administration route, and the period, but may be appropriately selected by a person skilled in the art.

In addition, the present disclosure provides a pharmaceutical composition for promoting pregnancy including beta core fragment hCG (βcf hCG) as an effective ingredient.

The term beta core fragment hCG of the present disclosure is as described above.

The pregnancy of the present disclosure is a process in which a fertilized egg implants on the uterine wall, receives nutrition from the mother, and develops into a fetus, and a normal pregnancy process proceeds in the order of fertilization, implantation, and fetal development.

The fertilization of the present disclosure means that a fertilized egg is formed when an egg ovulated from a woman's ovary meets a sperm in the upper part of the fallopian tube. An egg has the ability to survive and fertilize for 1 to 2 days after ovulation, and a sperm survives for 2 to 3 days in the uterus. In the case of sperm, it can survive for up to 1 week after ejaculation. Since sperm travels along the fallopian tube to the ovary within 2-3 hours after ejaculation, sperm generally first reaches the upper part of the fallopian tube, and when the egg ovulated from the ovary reaches this place, only one of the sperm combines with the egg to form a fertilized egg.

The implantation of the present disclosure refers to the phenomenon in which the fertilized egg formed by the meeting of the sperm and the egg in the upper part of the fallopian tube undergoes cleavage and moves to the uterus, becoming embedded in the uterine wall in the state of a blastocyst. The period after the fertilized egg is implanted is called pregnancy. The fertilized egg implanted in the uterus grows for about 9 months while receiving nutrients from the uterine wall and is then born.

The composition of the present disclosure may contain only the beta core fragment hCG, or may further contain a substance effective in promoting pregnancy as an active ingredient, and in addition to the above active ingredient, may further contain additional ingredients, that is, pharmaceutically acceptable or nutritionally acceptable carriers, excipients, diluents, or auxiliary ingredients, depending on the formulation, method of use, and purpose of use.

The composition can be used for promoting pregnancy and can be used in combination with surgery, radiation therapy, hormone therapy, chemotherapy, and biological response modifiers.

### [ADVANTAGEOUS EFFECTS OF THE INVENTION]

The present disclosure relates to a composition for treating infertility, which includes beta core fragment hCG (βcf hCG) as an active ingredient. The βcf hCG is identified to stimulate endometrial development in early pregnancy, is highly expressed in placental tissue, and is involved in maintaining fetal development in early pregnancy. The composition plays an important role in implantation and maintenance of early pregnancy by increasing the thickness of the endometrium, increasing the expression of genes necessary for uterine development, and promoting the growth of uterine-derived cell line. The composition can be usefully used to treat habitual miscarriage in female infertility, and to increase the success rate of pregnancy by improving the implantation rate of fertilized eggs in the process of in vitro fertilization.

### [DESCRIPTION OF THE DRAWINGS]

FIG. 1 is a diagram illustrating the main expression tissue of βcf hCG, and is a diagram showing that it is specifically expressed in placental tissue.
FIG. 2 is a diagram illustrating the change in endometrial thickness when βcf hCG is administered to the uterus of an experimental animal.
FIG. 3 is a diagram illustrating that the endometrial thickness of βcf hCG increased on the first and third days of administration compared to the control group (saline administration group) and the comparison group (highly purified intact hCG).
FIG. 4 is a diagram illustrating the result of observing the change in expression of a gene related to uterine receptive development and implantation in the uterus treated with βcf hCG.
FIG. 5 is a diagram illustrating the growth of cell line treated with βcf hCG at a concentration of 0.1 to 1 pmol/mL for 24 hours and 48 hours, compared to highly purified intact hCG.

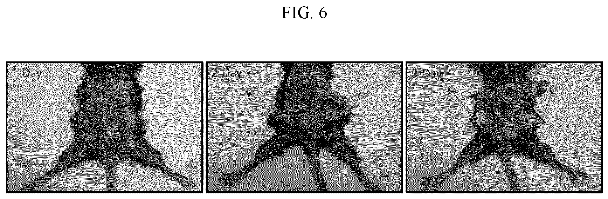

FIG. 6 is a diagram illustrating the result on days 1 to 3 after βcf hCG was administered into the mouse uterine cavity.
FIG. 7 is a diagram illustrating the results of measuring the cell viability of uterine cell line treated with βcf hCG.

### [BEST MODE]

Hereinafter, the contents of the present disclosure will be described in more detail through the following examples and experimental examples. However, the scope of the rights of the present disclosure is not limited to the following embodiments and experimental examples, and includes modifications of technical ideas equivalent thereto.

### Embodiment 1. Cell culture and βcf hCG treatment

Primary mouse endometrial cells obtained from uterine tissue of B6D2F1 mice are obtained. The extracted uterus is finely chopped and enzymatically dissociated in a mixture of Collagenase V (Sigma; C9263-100MG; 0.4 mg/ml) and dispase II (Sigma; D4693; 1.25 IU/ml) at 37°C for 1 hour. The cells are sequentially separated through a strainer (40 µm; SPL, 93040).

The filtrate containing the endometrial cells is collected by centrifugation and cultured with DMEM/F12 supplemented with 20% heat-inactivated FBS, 1% L-glutamine (Gibco, Grand Island, NY, USA), and 1% penicillin-streptomycin. Up to 10 passages are used in the experiment. For the analysis of the effect of βcf hCG, the cells are treated with βcf hCG at a concentration of 50 µg/100 µl for 2 to 72 hours.

### Embodiment 2. Intrauterine administration of βcf hCG to animal

The therapeutic effect analysis and evaluation of βcf hCG use are performed using 10-week-old B6D2F1 female and male mice (Orientbio, Gapyeong, Gyeonggi-do). All animal husbandry and experimental procedures are performed in accordance with the policies and regulations of the Institutional Animal Care and Use Committee. Mice are housed in a standard environment under controlled room temperature (20-22°C and 40-60% humidity) under 12-h light: 12-h dark conditions.

To evaluate the effect of βcf hCG on endometrial receptivity, female mice are randomly selected and βcf hCG (40 pmol/ml, 20 µl) is administered to one side of the mouse uterine cavity (FIG. 8). For the control group, saline injection is performed to the opposite side of the uterine horn. Both sides of the uterine horn are collected at the designated time points for further analysis. The βcf hCG dose applied in the experiment is selected based on clinical studies targeting infertile women. In many studies, 500 IU of hCG is injected intrauterinely during IVF-ET. This dose is converted to the animal-to-human dose conversion guideline and applied to the experiment.

### Embodiment 3. Histological analysis

Hematoxylin and Eosin (H&E) staining is performed to confirm morphological changes upon hCG administration. The obtained tissues are fixed with 4% paraformaldehyde and dehydrated using 80-100% ethanol and Histoclear (National diagnostics; HS-202). The tissues are then embedded vertically in paraffin.

The paraffin blocks are sectioned at 5 µm, and the sections are stained with hematoxylin (BioGNOST; HEMH-49/20) for 30 seconds and eosin (BioGNOST; EOYA-10-17/20) for 20 seconds. After mounting with Permount medium (Fisher chemical; 196934), images are captured using an Olympus CKX53 microscope (Olympus Life Science), and total gland number, endometrial area, and endometrial thickness are assessed using Image J.

### Embodiment 4. qRT-PCR analysis

Tissues are homogenized in 500 µl of labozol reagent (Cosmo; CMRZ001), and 1 µg of total RNA is converted to complementary DNA using SuperScript^{™} IV (invitrogen; 18091050). Amplification is performed on a CFX Connect Real-time PCR Detection System (Bio-Rad, Hercules, CA, USA) using SYBR Green (Roche, Basel, Switzerland).

A standard curve for each primer is generated by plotting the log value of the starting template amount against the cycle threshold obtained during amplification of each dilution showing an R2 value greater than 0.98 and an amplification efficiency of approximately 100%. All reactions are performed in triplicate.

### Embodiment 5. Statistical analysis

The comparison groups are analyzed by the unpaired Student's t-test for parametric distributions. For multiple comparisons, the general one-way ANOVA with Dunnett's multiple comparison test is used. In all cases, a value of p<0.05 is considered statistically significant.

### Experimental example 1. Identification of the main expression tissue of βcf hCG

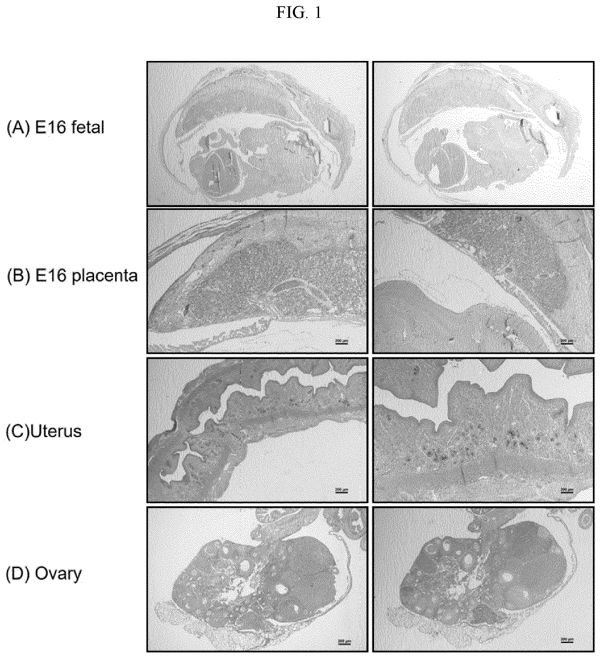

βcf hCG is identified to be highly specifically expressed in placental tissues compared to intact hCG in mouse fetuses on day 16 after pregnancy (E16 mouse) (FIG. 1).

βcf hCG is shown to be associated with changes in the expression of epithelial granular tissue involved in implantation of the fertilized egg in the endometrial tissue of the non-pregnant uterus.

This suggests that histologically, βcf hCG stimulates the development of the endometrium in the early stage of pregnancy and is expressed in large quantities in the placental tissue during pregnancy, thereby participating in the maintenance of fetal development in the early stage of pregnancy.

### Experimental Example 2. Endometrial Development by βcf hCG

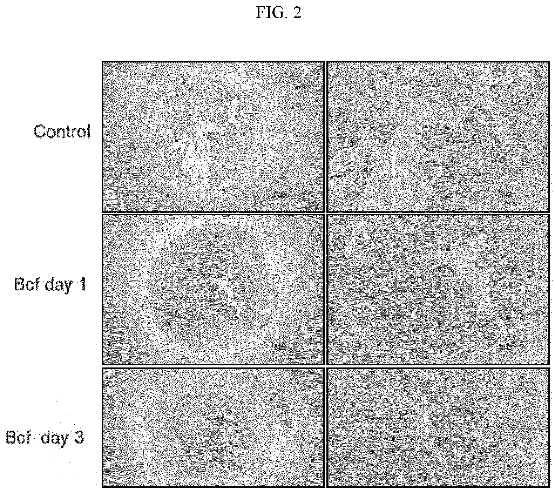

When 200 µL βcf hCG (40 pmol/mL) is artificially administered intravaginally to the uterus of experimental animals, the thickness of the endometrium significantly increased compared to the control group (FIG. 2).

In addition, it is identified that granulosa cell activity significantly increased in the endometrium treated with βcf hCG.

Granulosa cells activate aromatase by FSH (follicle-stimulating hormone), which promotes estrogen biosynthesis from androstenedione produced by LH (luteinizing hormone).

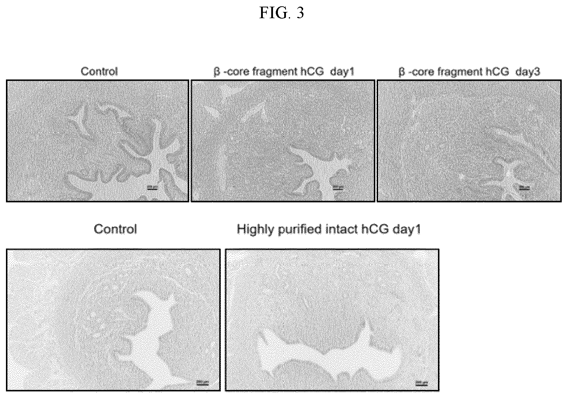

As represented in Table 1, Table 2, FIGS. 2 and 3, the thickness of the endometrium of the group treated with βcf hCG is identified to increase by about 19.72 to 23% or more compared to the control group. On the other hand, the group treated with intact hCG showed a 15% increase compared to the control group.

**[Table 1]**

| Type | Cases (n) | Uterine gland number (n) | Uterine gland number Increase percentage (%) | Uterus area increase percentage(%) |
|---|---|---|---|---|
| Control | 2 | 49.5 | - | - |
| β-core fragment hCG day1 | 2 | 82.5 | 66.67% | 19.72% |
| β-core fragment hCG day3 | 2 | 51 | 3.03% | 1 23.03% |

**[Table 2]**

| Type | Cases(n) | Uterine gland number (n) | Uterine gland Increase percentage (%) | Uterus area increase percentage (%) |
|---|---|---|---|---|
| I Control | 2 | 41.5 | - | - |
| Highly purified intact hCG day1 | 2 | 37.5 | -9.64 | 15.49% |

### Experimental Example 3. Analysis of Gene Expression of Factors Necessary for Uterine Development by βcf hCG

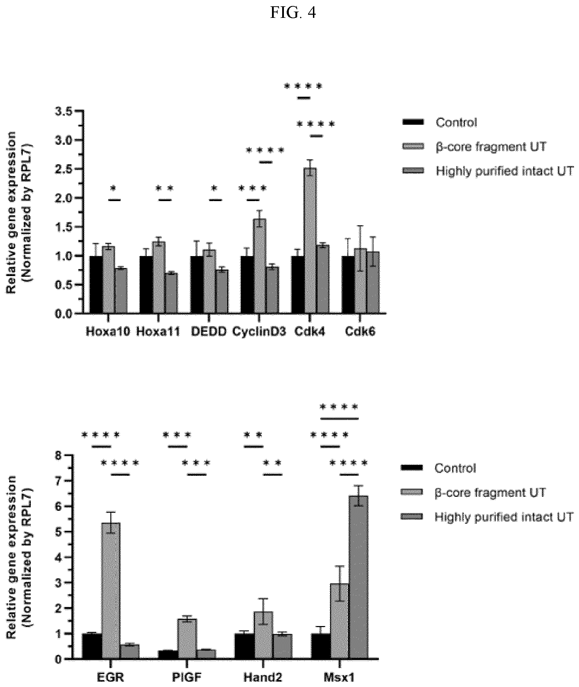

It is identified that a gene promoting the growth of endometrial cells is expressed in the uterus treated with βcf hCG. It is identified that the expression of the gene related to uterine receptive development and implantation increased in the uterus treated with βcf hCG (FIG. 4).

Specifically, HOXA10 plays a role in improving endometrial receptivity by regulating cholesterol synthesis in endometrial stromal cells. HOXA11 is involved in endometrial proliferation, differentiation, and embryonic development. DEDD plays an essential role in forming an appropriate uterine environment for early pregnancy. Cyclin D3 and CDK4/6 are closely related to cell cycle regulation for endometrial cell proliferation. HAND2 plays an important role in forming an appropriate implantation environment for pregnancy, and it forms a uterine environment that is sensitized to progesterone when implantation begins. PIGF affects embryo implantation and development, and induction of endometrial development. EGR is an immediate early gene important for cell growth and differentiation, and is expressed in response to stimulation rapidly. MSX1 controls uterine receptivity by regulating uterine epithelial function at the time of embryo implantation.

As a result, the expression of the gene is identified to increase in the uterus treated with βcf hCG. This indicates that it will have an important effect on uterine receptive development and implantation. In addition, it indicates that it can contribute to successful embryo implantation by improving the adaptability of the uterine environment in the early stage of pregnancy.

### Experimental Example 4. Growth of cell line treated with βcf hCG in placental and endometrial cell line

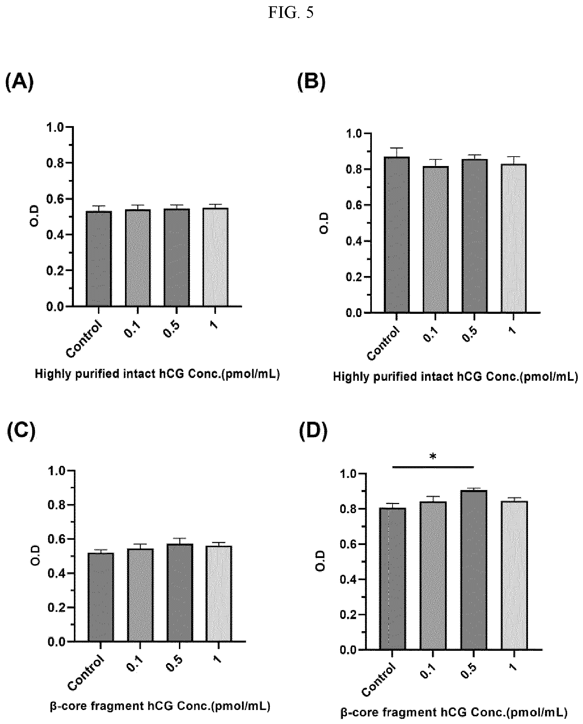

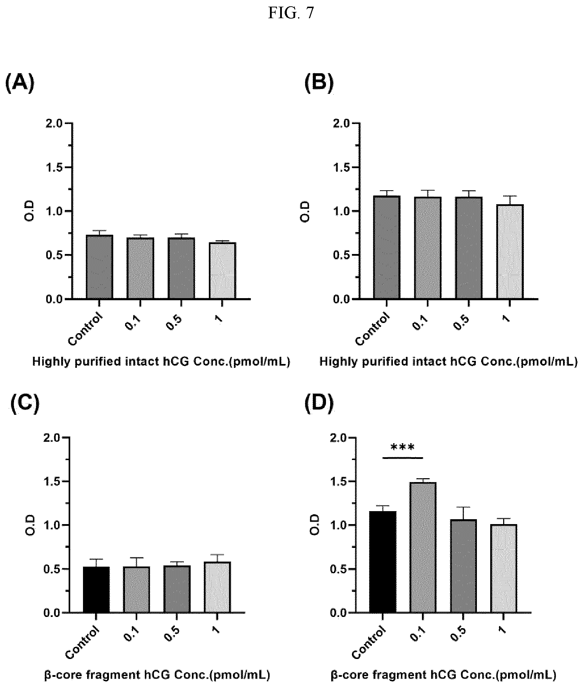

When βcf hCG is applied to placental-derived cell line at a concentration of 0.1 to 1 pmol/mL for 24 and 48 hours, it is identified that the growth of the cell line increased compared to the control group (FIG. 5). As a result of treating with βcf hCG, it is identified that the cell viability of the endometrial cell line also significantly increased (FIG. 7).

Through these results, it is shown that βcf hCG plays an important role in the early implantation and maintenance of pregnancy through the development of the endometrium. This can be developed as a treatment to improve habitual miscarriage in female infertility, and can be usefully applied to increase the pregnancy success rate by improving the implantation rate of fertilized eggs during in vitro fertilization.

Although the embodiments of the present disclosure have been described above, the present disclosure is not limited to the embodiments, but can be manufactured in various different forms, and those skilled in the art to which the present disclosure pertains will understand that the present disclosure can be implemented in other specific forms without changing the technical idea or essential features of the present disclosure. Therefore, it should be understood that the embodiments described above are exemplary in all aspects and not limiting.

## Claims

1. A pharmaceutical composition for preventing or treating infertility, comprising beta core fragment hCG (βcf hCG) as an active ingredient.

2. The pharmaceutical composition according to claim 1,
wherein the beta core fragment hCG comprises 6^{th} to 40^{th} amino acids and 55^{th} to 92^{th} amino acids of β-hCG, and an amino acid sequence of the beta core fragment hCG comprises sequence numbers 2 and 3.

3. The pharmaceutical composition according to claim 1,
wherein the beta core fragment hCG is secreted into urine of a pregnant woman or secreted from placenta and corpus luteum of an ovary.

4. The pharmaceutical composition according to claim 1,
wherein the composition is administered within 50 to 90 hours before embryo implantation.

5. The pharmaceutical composition according to claim 4,
wherein the administration comprises an intrauterine administration.

6. The pharmaceutical composition according to claim 1,
wherein the composition increases a thickness of an endometrium.

7. The pharmaceutical composition according to claim 1,
wherein the composition promotes a proliferation of an endometrial cell.

8. The pharmaceutical composition according to claim 1,
wherein the composition increases an expression of a gene related to uterine receptive development and implantation.

9. The pharmaceutical composition according to claim 8,
wherein the gene comprises HOXA10, HOXA11, DEDD, Cyclin D3, CDK4/6, EGR, PIGF, HAND2 or MSX1.

10. The pharmaceutical composition according to claim 1,
wherein the infertility is at least one selected from a group consisting of nonimplantation of ovum, female infertility associated with anovulation, female infertility of tubal origin, female infertility of uterine origin, female infertility of cervical origin, and female infertility associated with male factors.

11. A method for preventing or treating infertility, comprising administering the pharmaceutical composition of claim 1 into a uterus.

12. The method according to claim 11,
wherein the method is administered to an animal other than a human.

13. A pharmaceutical composition for promoting pregnancy comprising beta core fragment hCG (βcf hCG) as an active ingredient.
